# EUROPEAN PATENT SPECIFICATION

(11) **EP 3 297 587 B1**
(45) Date of publication and mention of the grant of the patent: **23.03.2022**
(21) Application number: 16724945.7
(22) Date of filing: 19.05.2016
(51) Int. Cl.: A61F 7/03, A61K 9/70, A61M 5/142, A61K 9/00, A61M 5/42, A61K 31/04, A61K 31/167, A61K 31/245, A61F 7/00, A61F 7/02

(54) **INTRAVENOUS ACCESS DEVICE**
INTRAVENÖSE ZUGANGSVORRICHTUNG
DISPOSITIF D'ACCÈS INTRAVEINEUX

(30) Priority: 21.05.2015 GB 201508720
(43) Date of publication of application: 28.03.2018
(73) Proprietor: Gateway Devices Limited, Ealing London W5 4HU (GB)
(72) Inventor: BATOOL, Zainab, Birmingham West Midlands B29 6EH (GB)
(74) Representative: Barker Brettell LLP
(86) International application number: PCT/GB2016/051444
(87) International publication number: WO 2016/185214

(56) References cited:
- US-A1- 2010 234 828
- US-A1- 2012 078 223
- US-A1- 2013 337 031

## Description

The present invention relates to a device for application to the skin of a patient in order to facilitate intravenous access.

### Background to the invention

Obtaining intravenous access, whether via cannulation (for the administration of intravenous fluids, medication or blood transfusion) or venepuncture (blood sampling for the purpose of laboratory testing), is the most commonly performed medical procedure. Insertion of intravenous access devices (cannulas or needles) by clinicians into human veins is carried out for almost all patients accessing hospital services, both at the point of presentation as well as at frequent intervals during admission.

A cannula is a device used to gain peripheral venous access for the administration of intravenous therapy. Cannulas are normally used to access to peripheral veins in a patient's hands, feet, inner elbow or forearm.

The cannula remains in situ for as long as the course of treatment is required. A cannula is made up of a needle surrounded by a plastic sheath. The needle is approximately 3 cm in length, with various diameters being available - a suitable size will depend on the size of the vein and the speed with which the intravenous fluid must be given. The needle is removed after insertion, but the plastic sheath remains in situ. Insertion of a cannula thus requires a straight stretch of vein to be seen or felt, and thus are often more difficult to insert than a simple needle for blood sampling.

Cannulas should be replaced about every 72 hours for infection control purposes. Therefore long term inpatients will have cannulas inserted on a regular basis.

Cannulation is typically accomplished by placing a rubber tourniquet around a patient's limb, for example a forearm, proximal to the planned point of access. The tourniquet causes compression of the superficial veins without compressing the associated arteries. Therefore, the blood is pumped through the arteries past the tourniquet into the distal extremity. Since the veins are compressed, the blood is prevented from returning to the heart. The veins typically dilate due to the increased intravascular pressure and are thus more visible and easier to access with the needle or cannula. Once the dilated vein is identified, the skin is cleaned. The needle or cannula is then inserted into the dilated vein.

It is often notoriously difficult to gain venous access; junior doctors are often delegated this task, and this forms a huge and often frustrating proportion of their workload. Venous access is particularly difficult to accomplish in various classes of patient, namely in infants and children, overweight patients, patients with darker skin, patients who have oedematous limbs, drug abusers, patients receiving chemotherapy for cancer and generally in patients who have blood taken on a regular basis. In very sick patients, peripheral veins 'shut down', thus making cannulation even more difficult, in a situation where it would be most urgent. Additionally, patients can be difficult to cannulate if they are cold, frightened or dehydrated. This commonly occurs in patients that are injured or are about to undergo surgery. In these situations, veins are actively constricted by the sympathetic nervous system and, therefore will be difficult to visualise or will not dilate in response to an increase in intravenous pressure. Frequently, the application of a tourniquet is not enough to cause the veins to visibly dilate.

Multiple attempts at cannulation or venepuncture are associated with increased cost of materials, pain and bruising for the patient, increased risk of infection, increased risk of extravasation (leakage of medication into the skin tissues), the risk of 'using up' veins in case of an emergency, and extensive use of doctor/nurse staff time.

In the current NHS system in the UK the average number of attempts required to insert each cannula is 2.84. Therefore in NHS admissions only, for the patient to receive their first cannula, an average of 34.5 million cannulas are used.

Under current UK NHS practice, if despite multiple attempts (three attempts is deemed 'acceptable') the initial medical practitioner is unable to cannulate, the process is escalated up a chain. The chain is from a specialist nurse to a junior doctor to a senior house officer to the registrar to an anaesthetist with the use of an ultrasound scan. If this too fails, a higher risk 'central line' must be inserted surgically. Each attempt for cannulation can take about 10 to 20 minutes for each medical practitioner involved. Thus this is a substantial use of medical practitioner time, which undoubtedly could be better used for patient care.

Electric heating pads and warming blankets that are placed surrounding the limb of a patient to reduce vasoconstriction and dilate veins are known. For example, US 2007/0068932 A1 discloses an electric heater having a temperature control for heating the limb of a patient. Such devices have been contemplated for use in assisting with venous access.

However, these types of heating device require plugging in to a source of electricity and so are not portable or applicable to most emergency settings.

A major concern with these, however, is undoubtedly the associated cost, which is a serious deterrent to national health systems that are always seeking to minimise costs within strict budgets. Hence no such device is currently used widely in any national health service.

In addition, such electric heating devices are difficult to wrap snuggly around the forearm and therefore typically do not maintain good contact with the skin for optimal conductive heat transfer.

Also, such devices that warm patient's limbs are bulky and cover a significant part of the limb. Therefore they cannot be used in patients having an intravenous access device already inserted in that limb. They generally comprise large pads which are wrapped around most or all of a patient's limb - e.g. all of the forearm and hand - and therefore are relatively cumbersome. It can take time to ensure that the device is fitted correctly. There can be difficulties if the patient has wounds or trauma in that limb where the vein to be cannulated is located.

Staff may also have to be trained more extensively in the use of these devices.

Additionally electric heating pads and warming blankets covering a patient's limb can be uncomfortable to wear and do not allow the patient to move that limb while the device is fitted.

Furthermore, hospitals need to have different sizes of these devices so they can be used in patients with shorter limbs, such children. This also makes the device a more expensive and time consuming option.

Thus, the prior art generally describes devices that are not ideal solutions in terms of cost, practicality and efficiency. The prior art devices cannot be used to heat just a specific area of the patient's limb or a specific vein segment into which the intravenous access device (e.g. cannula, needle or catheter) is going to be inserted.

In view of the above, there is still a need for a device which addresses one or more of the above issues.

There is, in particular, a need for a device which efficiently improves vein access, is simple to apply, can be used just in a specific region of an extremity, and achieves a rapid dilation and/or improved vein visibility in the planned point of insertion in a time/cost-efficient manner.

For example, it would be desirable to provide a "one size fits all" device which can be used for all patients, and at various sites on the body.

It would be desirable to provide a device that reduces the number of attempts required to cannulate, which would therefore make the process quicker and reduce the number of occasions on which multiple medical practitioners need to be involved and the number of occasions a higher risk 'central line' must be inserted surgically.

US 2010/234828 describes methods and compositions for locally increasing arterial diameter and providing local anesthesia in a subject. The methods include applying to a skin site of the subject a topical composition that includes a vasodilation agent and an anesthetic agent in a manner sufficient for locally increasing arterial diameter and providing local anesthesia in the subject. Topical compositions that find use in performing the subject methods are also described.

US 2012/078223 describes a warming garment and method of making and using the garment. The warming garment may have an inner lining and an outer lining which may form an annular space in which an exothermic composition that is activated upon exposure to oxygen may be disposed. When worn, the warming garment may aid in inducing vasodilation upon a subject's limb through the emanation of heat from the exothermic composition. Once vasodilation is achieved, venipuncture or catheter insertion procedures may be properly initiated.

US 2013/337031 describes topical formulations, transdermal systems, and related methods. The topical formulation may include a local anesthetic, a first compound, and a second compound. The first compound and second compound are different and each is selected from the group consisting of N-lauroyl sarcosine, sodium octyl sulfate, methyl laurate, isopropyl myristate, oleic acid, glyceryl oleate, and sodium lauryl sulfoacetate.

### Summary of the invention

The present invention provides a device for application to the skin of a patient to facilitate intravenous access, the device having an upper surface and a lower surface, wherein the lower surface is suitable for contacting the skin of the patient at the location of a vein to be accessed, the device comprising a heat patch, an organic nitrate vasodilator agent and an amino based anaesthetic agent which is an amino ester or amino amide, wherein the heat patch is activated by the application of pressure and in this regard can be flexed or pressed to initiate an exothermic chemical reaction to heat the patch to a temperature of from 50 to 55 degrees Celsius; wherein the device is configured and arranged such that in use the device can be activated by the application of pressure to the patch so as to initiate an exothermic chemical reaction in the heat patch which heats the patch to a temperature of from 50 to 55 degrees Celsius for a period of time of one minute or more, and wherein the device is configured and arranged such that when the device is applied to the skin of the patient, with the lower surface contacting the skin of the patient, the vasodilator agent and the anaesthetic agent are released from the lower side of the device onto the patient's skin at the location of a vein to be accessed, due to (i) the vasodilator agent and anaesthetic agent each being provided as a coating on the lower surface, or (ii) the device including a reservoir that releasably contains both the vasodilator agent and the anaesthetic agent; or (iii) the device including one reservoir that releasably contains the vasodilator agent and one reservoir that releasably contains the anaesthetic agent.

The device may be suitable for application to the skin of a patient at any suitable location. Preferred locations for accessing veins are the patient's limbs, i.e. their arms and legs. Particularly preferred locations include, but are not limited to, the hands, the feet and the inner elbows (the antecubital fossae of the elbows).

The activation of the device by the application of pressure to the patch, so as to initiate an exothermic chemical reaction in the heat patch which heats the patch, may be by pressing the patch or by flexing the patch. Heat patches where an exothermic chemical reaction is initiated by pressing or flexing the patch are known in the art. However, the present invention presents a new use for these patches, as it has been found, unexpectedly, that the time taken to cannulate and the number of attempts to reach successful cannulation can be significantly reduced by the use of this type of heat source heating to a temperature of from 50 to 55 degrees Celsius when used in combination with two specific active agents.

The present invention provides a device to aid intravenous access, which can be used in preparation for venepuncture (e.g. insertion of an intravenous access device such as a needle, catheter or cannula).

Surprisingly, the application and activation of the present device to the skin of the patient at the location of a vein to be accessed results in a significant reduction in the number of attempts required to achieve successful cannulation. As noted above, the number of attempts is a key criterion within current systems. If this number can be reduced, the process becomes much quicker, saving time for medical practitioners. The number of occasions on which the cannulation would need to be escalated up to a more senior practitioner in the chain would be reduced.

Surprisingly, the application of the present device to the skin of the patient at the location of a vein to be accessed results in a significant increase in the rate of successful cannulation. Therefore the number of occasions on which the cannulation would need to be escalated up to a more senior practitioner in the chain would be reduced. The number of time where a higher risk 'central line' must be inserted surgically would be reduced.

Surprisingly, the application of the present device to the skin of the patient at the location of a vein to be accessed results in the time taken to achieve successful cannula insertion being significantly reduced. If this time can be reduced, the process becomes much quicker and the time of medical practitioners is freed up.

Further, the application of the device of the present invention to an area of skin on a patient's limb is more straightforward and faster than fitting tourniquets or typical electric heating pads or blankets which need to be wrapped around the limb of the patient. Indeed, such prior art devices cannot be fitted around the limb of a patient already having an intravenous access device (for example a cannula) inserted in their limb but needing additional cannulation in that same limb. In these situations additional cannulation is even more difficult, because the patient may have their veins constricted due the presence of the already-inserted cannula, and so their veins may not dilate in response to an increase in intravenous pressure.

In contrast, the device of the present invention can be applied locally, in a specific area of the skin where there is a vein to be accessed, and achieves a localized and highly effective vasodilatation when applied.

The device therefore provides significant benefits for the medical practitioner who needs to achieve intravenous access, e.g. for cannulation.

The device of the present invention is also beneficial in that it allows the medical practitioner to reduce the time spent in setting up the equipment and in achieving successful intravenous access. The application of the device of the invention to a localized area of a patient's limb does not require a complex fitting, or the selection of a suitable size of product, or the need to have a power source available so that the device can be connected to a source of electricity.

The medical practitioner or an assistant simply needs to take one of the "one size fits all" devices according to the invention, apply it to the skin of the patient at the location of the selected vein of interest, and activate the device by pressure.

Alternatively, the medical practitioner or an assistant can take one of the "one size fits all" devices according to the invention, activate the device by pressure, and then apply it to the skin of the patient at the location of the selected vein of interest. As long as the practitioner does not delay applying the activated device to the patient's skin for too long there is no reason why the activation cannot be carried out before the device is applied to the skin. Heat patches that can be activated by pressure to initiate an exothermic reaction will normally only generate heat for a certain length of time, for example from 3 to 10 minutes, although the time frame can vary. If the device is activated before it is applied to the patient's skin, then in accordance with the present invention the device must be applied to the patient's skin while the patch is still at a temperature of from 50 to 55 degrees Celsius.

For example, it might be recommended that if the device is activated before it is applied to the patient's skin, the device must be applied to the patient's skin within two or three minutes of activation, preferably within 90 seconds of activation, such as from 1 second to 90 seconds after activation, more preferably within a minute of activation, such as from 1 second to 60 seconds after activation.

Therefore the disclosure provides, but does not claim, a method for facilitating intravenous access to a selected vein in a patient, the method comprising:
i) placing the device of the first aspect of the invention onto the skin of the patient at a location proximal to the selected vein;
ii) activating the device so as to initiate the exothermic chemical reaction in the heat patch to heat the patch to a temperature of from 50 to 55 degrees Celsius;
   wherein steps i) and ii) can be carried out in any order,
   and then
iii) leaving the device on the skin of the patient for a time period of from one minute to ten minutes;
   and then
iv) removing the device from the patient's skin.

Intravenous access is facilitated by carrying out these steps. These steps can be carried out by a medical practitioner, but equally could be carried out by an assistant whilst waiting for the doctor to arrive, for example, as they do not require medical expertise.

A medical practitioner can then carry out a method of intravenous access, for example cannulation, in relation to the selected vein.

The unclaimed method of the disclosure is also beneficial in that while the device is on the skin of the patient in step iii) the medical practitioner or an assistant can prepare the equipment for cannulation. The time taken to prepare equipment for cannulation is, on average, about one to ten minutes, and thus the equipment can be prepared as the patient's vein is being prepared. Clearly the device of the first aspect of the invention should be placed onto the skin of the patient at a location as close as possible to place at which it is intended that the selected vein will be accessed (e.g., cannulated).

Preferably steps i) and ii) are carried out within three minutes of each other, more preferably within two minutes of each other, such as within 90 seconds of each other, most preferably within a minute of each other.

The use of the device will cause dimensional and coloration changes which serve to make the vein more accessible before insertion of an intravenous access device, such as a cannula or needle or catheter.

In a second aspect the present invention provides a kit comprising the device of the first aspect and an intravenous access device, such as a cannula or needle or catheter.

In one embodiment the intravenous access device is a cannula.

### Detailed description of the invention

The device for topical application of the present invention comprises a heat patch. This heat patch is activated by the application of pressure. In this regard, it can be flexed or pressed to initiate an exothermic chemical reaction to heat the patch to a temperature of from 50 to 55 degrees Celsius.

In one embodiment of the present invention, the exothermic chemical reaction heats the patch to a temperature of from 50 to 54 degrees Celsius, such as from 50 to 53 degrees Celsius or from 50 to 52 degrees Celsius. In one embodiment of the present invention, the exothermic chemical reaction heats the patch to a temperature of from 51 to 55 degrees Celsius, such as from 51 to 54 degrees Celsius or from 51 to 53 degrees Celsius. In one embodiment of the present invention, the exothermic chemical reaction heats the patch to a temperature of from 50 to 54 degrees Celsius, such as from 51 to 54 degrees Celsius or from 52 to 54 degrees Celsius.

It is preferred the exothermic chemical reaction heats the patch to a temperature of from 50 to 55 degrees Celsius for a period of time of two minutes or more, such as from two to 15 minutes or more, more preferably for a period of time of three minutes or more, such as from three to 12 minutes or more, most preferably for a period of time of four minutes or more, such as from four to 10 minutes, or more. In one embodiment the exothermic chemical reaction heats the patch to a temperature of from 50 to 55 degrees Celsius for a period of time of five minutes or more, such as from 5 to 10 minutes.

In one embodiment, the exothermic chemical reaction heats the patch to a temperature of from 50 to 55 degrees Celsius for less than 15 minutes, such as 12 minutes or less, or 10 minutes or less. In one embodiment, the exothermic chemical reaction heats the patch to a temperature of from 50 to 55 degrees Celsius for from 3 to 15 minutes or from 3 to 12 minutes, such as from 3 to 10 minutes.

In one embodiment, when the heat patch is activated to initiate the exothermic chemical reaction, the lower surface of the device is, as a consequence, heated to a temperature of from 40 to 46 degrees Celsius. For example, the lower surface of the device may be heated to a temperature of from 40 to 45 degrees Celsius or from 40 to 44 degrees Celsius or from 40 to 43 degrees Celsius. In one embodiment, the lower surface of the device may be heated to a temperature of from 41 to 46 degrees Celsius or from 41 to 45 degrees Celsius or from 41 to 44 degrees Celsius. In one embodiment, the lower surface of the device may be heated to a temperature of from 42 to 46 degrees Celsius or from 42 to 45 degrees Celsius or from 42 to 44 degrees Celsius.

The temperature at the lower surface of the device is affected by the temperature to which the heat patch is heated by the exothermic reaction, the distance between the heat patch and the lower surface, and the conductivity of the materials in the space between the heat patch and the lower surface. These can be selected appropriately by the skilled person to ensure that the required temperature is achieved.

In one embodiment of the invention the distance between the heat patch and the lower surface is from 0m to 10mm, such as from 0mm to 5mm or from 0mm to 2mm, e.g. about 0mm or about 1mm.

It is an important aspect of the invention that the exothermic chemical reaction heats the patch to a temperature of from 50 to 55 degrees Celsius. This contrasts with prior heat devices that taught the application of heat to induce vasodilation, which used heating to temperatures around 36 to 42 degrees Celsius.

It has been found, unexpectedly, that the time taken to cannulate and the number of attempts to reach successful cannulation can be significantly reduced by the use of a heat patch heating to a higher temperature, specifically of from 50 to 55 degrees Celsius, when used in combination with two specific active agents. This is therefore a specific combination of features that was not taught in the art and that has a technical effect that was not predicted. As can be seen from the examples, the results are a notable improvement in terms of the time taken to cannulate and the number of attempts to reach successful cannulation can be significantly reduced.

In one embodiment the exothermic chemical reaction to heat the patch is an oxidation reaction or a crystallization reaction. Heat patches capable of an exothermic oxidation reaction typically comprise a chemical composition, which is exothermically reactive in the presence of air. Heat patches capable of an exothermic crystallization reaction typically comprise a container for a supercooled aqueous salt solution which, when activated, releases heat. Heat packs based on an exothermic crystallization reaction are described in, for example, US patent 4,077,390. The teachings of that document can be read and understood with regard to implementing the type of heat patch that can be used in the invention.

In one embodiment of the present invention the exothermic chemical reaction to heat the patch is an oxidation reaction and the heat patch comprises a chemical composition which is exothermically reactive in the presence of air. Suitable compositions typically comprise a mixture of iron, carbon, a chloride or sulfate salt, and water which generate heat on exposure to air. Other suitable compositions comprise a moist particulate mixture of carbon powder, iron powder, vermiculite, and a salt-water solution.

In one preferred embodiment of the present invention the exothermic chemical reaction to heat the patch is a crystallization reaction and the heat patch comprises a container that contains a supercooled aqueous salt solution which, when activated, releases heat.

These solutions are supersaturated solutions of the salt in water which can be triggered to crystallize, e.g. by the release of small crystals into the solution which then act as nucleation sites (see Rogerson, M. A. and Cardoso, S. S. S. (2003), Solidification in heat packs: III. Metallic trigger. AIChE J., 49: 522-529). Due to the fact that the solution is supersaturated, the solution will crystallize suddenly, releasing the energy of the crystal lattice.

Suitable salt solutions include sodium acetate, lead acetate, calcium nitrate tetrahydrate, sodium pyrophosphate or sodium thiosulfate in the form of a supercooled aqueous solution. Sodium acetate may be preferred because it is generally harmless to humans.

The salt solution is made by dissolving the salt in suitable amount of water. The amount of salt to be utilized should permit the salt solution to be supercooled to at least the ambient temperature at which the heat patch is intended to be utilized.

The supercooled aqueous salt solution may optionally further include one or more additional agents selected from the group consisting of: viscosity increasing agents, gelling agents and thickening agents.

A trigger to initiate crystallization is included in the crystallization reaction heat patch. Many triggers have been used in prior art heat packs and may be used in the present heat patch. These are often substrates that can harbour small crystals which can then be released into the solution when desired, e.g. by pressing or flexing the substrate, and which then act as nucleation sites to cause the crystallization and release the energy of the crystal lattice.

The trigger may be formed from any suitable material that can reliably and repeatedly initiate crystallization of the supercooled solution. For example, the trigger may be a metal or mineral strip or disc. Metal strips or discs are commonly used in this regard and the metal may optionally be an alloy. The trigger may, for example, be formed of flint, garnet, aluminium oxide, silicon carbide, alumina-zirconia, chromium oxide, ceramic aluminium oxide, ferrous material, phosphor bronze, beryllium copper and the like. In one embodiment, the trigger is a metal strip or disc formed from ferrous material. In one embodiment the trigger is a stainless steel strip or disc.

When the trigger is manipulated by the application of pressure, for example by pressing or by flexing the trigger, this initiates crystallization.

Chemical triggering means may also be used. These are also known in the art.

In one particular embodiment the heat patch comprises a container, a supercooled salt solution within said container and a trigger for initiating crystallization of said supercooled salt solution, said trigger being in contact with said solution. Preferably most or all of the trigger is immersed in the supercooled salt solution.

When the trigger is manipulated by the application of pressure, e.g. when the trigger is pressed or flexed, this causes the solution to crystallize with ensuing evolution of heat to a temperature of from 50 to 55 degrees Celsius. The application of pressure may involve the trigger being squeezed or bent or even snapped.

Preferably the supercoolable aqueous solution is a sodium acetate solution.

The device also comprises organic nitrate vasodilator agent and amino based anaesthetic agent. Both agents must be suitable for topical application, because in the invention the vasodilator agent and the anaesthetic agent are released from the lower side of the device onto the patient's skin at the location of a vein to be accessed.

In one embodiment the organic nitrate vasodilator agent is selected from the group consisting of glyceryl trinitrate, isosorbide dinitrate, isosorbide mononitrate, erythritol tetranitrate, pentaerythritol tetranitrate, and combinations thereof.

Preferably the organic nitrate vasodilator agent is selected from the group consisting of glyceryl trinitrate, isosorbide dinitrate, isosorbide mononitrate, and combinations thereof.

More preferably the organic nitrate vasodilator agent is glyceryl trinitrate (also known as nitroglycerine).

In one embodiment, the device comprises from 1 mg to 100 mg of organic nitrate vasodilator agent, such as from 2mg to 50mg or from 5mg to 25mg.

In one embodiment, the device comprises from 1 mg to 50 mg of organic nitrate vasodilator agent, such from 1 mg to 45 mg, or from 1 mg to 40mg, or from 1 mg to 35 mg. It may be that the amount of organic nitrate vasodilator agent in the device is from 1 mg to 45 mg, such as from 2 mg to 40 mg, e.g. from 2 mg to 35 mg. In one embodiment the amount of organic nitrate vasodilator agent in the device is from 3 mg to 30 mg, such as from 3 mg to 25 mg or from 4mg to 20mg.

In one embodiment, the amount of organic nitrate vasodilator agent in the device is from 5mg to 20mg, such as from 5mg to 15mg.

The device of the present invention also includes an anaesthetic agent. Anaesthetic agents are of course known in the art. For example, US2013/037031 relates to the application of anaesthetic to a subject experiencing pain and the use of molecular penetration enhancers to aid delivery of the anaesthetic. This document optionally may heat the skin to a temperature of 35 to 47 degrees Celsius (preferably 38 to 42, or 36 to 40, degrees Celsius) to further assist the pain relief. The document teaches the application of its system to the patient's skin for at least 15 minutes and over time frames such as 2 hours, 4 hours, 12 hours and 24 hours. In such uses the anaesthetic is therefore applied to the subject experiencing pain for a prolonged period, because the aim is to reduce the subject's pain.

In contrast, the device of the present invention is intended to be applied to the patient's skin for relatively short time frames, in preparation for intravenous access, whether via cannulation (for the administration of intravenous fluids, medication or blood transfusion) or venepuncture (for blood sampling for the purpose of laboratory testing). For example, the device may be applied for a time period of from one minute to ten minutes, such as from three to ten minutes.

The present device is not a pain relief treatment. Instead, the device uses the combination of the organic nitrate vasodilator agent and the amino based anaesthetic agent and the exothermic chemical reaction which heats the patch to a temperature of from 50 to 55 degrees Celsius to prepare the skin for intravenous access, e.g. cannulation. The device is not designed for treating pain in a subject, whereby prolonged treatment timeframes would be used and would be necessary to ensure pain relief for the subject. Instead, the device provides a short (e.g. 10 minutes or less) preparation treatment that improves the subsequent intravenous access, e.g. cannulation. Unexpectedly, when this preparation treatment is used, the time taken to cannulate and the number of attempts to reach successful cannulation can be significantly reduced. As can be seen from the examples, the results are a notable improvement in terms of the time taken to cannulate and the number of attempts to reach successful cannulation can be significantly reduced.

The benefit of using the combination of the organic nitrate vasodilator agent and the amino based anaesthetic agent and the exothermic chemical reaction which heats the patch to a temperature of from 50 to 55 degrees Celsius were not recognised in the art. There is no teaching that this combination of three elements could particularly effectively prepare the skin for intravenous access, e.g. cannulation.

As noted above, the anaesthetic agent used in the present invention is an amino ester or amino amide that can be applied topically. In one embodiment the agent is an amino ester anaesthetic agent selected from the group consisting of procaine, chloroprocaine, tetracaine, cocaine, and benzocaine. In one embodiment the agent is an amino amide anaesthetic agent selected from the group consisting of dibucaine, lidocaine, mepivacaine, prilocaine, bupivacaine, levobupivacaine, ropivacaine, articaine, and etidocain.

Suitably, therefore, the amino ester or amino amide anaesthetic agent is selected from the group consisting of procaine, chloroprocaine, tetracaine, cocaine, benzocaine, dibucaine, lidocaine, mepivacaine, prilocaine, bupivacaine, levobupivacaine, ropivacaine, articaine, and etidocain, and combinations thereof.

In one embodiment the anaesthetic agent is selected from the group consisting of lidocaine, prilocaine, tetracaine, prilocaine, dibucaine, benzocaine and combinations thereof.

Preferably, the anaesthetic agent is lidocaine, prilocaine or tetracaine or a combination thereof. It may, for example, be a mixture of lidocaine and prilocaine, or it may be tetracaine.

In one embodiment, the device comprises two or more amino based anaesthetic agents. There may be two (or more) amino esters, or there may be two (or more) amino amides, or there may be one (or more) amino ester and one (or more) amino amide.

In one embodiment, the device comprises a mixture of two anaesthetic agents, in a ratio of from 2:1 to 1:2, e.g. a ratio of about 1:1.

In one embodiment, the device comprises a mixture of lidocaine and prilocaine, in a ratio of from 2:1 to 1:2, such as a 1:1 mixture of lidocaine and prilocaine.

In one embodiment, the device comprises from 1 mg to 100 mg of amino based anaesthetic agent which is an amino ester or amino amide, such as from 2mg to 50mg or from 5mg to 25mg.

In one embodiment, the device comprises from 2 mg to 80 mg of amino based anaesthetic agent which is an amino ester or amino amide, such from 3 mg to 75 mg, or from 4 mg to 70mg, or from 5 mg to 65 mg. It may be that the amount of amino based anaesthetic agent which is an amino ester or amino amide in the device is from 5 mg to 60 mg, such from 5 mg to 55 mg, or from 5 mg to 50 mg, or from 5 mg to 45 mg, e.g. from 5mg to 40mg.

In one embodiment the amount of amino based anaesthetic agent which is an amino ester or amino amide in the device is from 6 mg to 60 mg, such as from 7 mg to 55 mg, or from 8 mg to 50 mg, or from 9mg to 45mg.

In one embodiment, the amount of amino based anaesthetic agent which is an amino ester or amino amide in the device is from 10 mg to 40 mg, such as from 10mg to 30mg.

Preferably the organic nitrate vasodilator agent is selected from the group consisting of glyceryl trinitrate, isosorbide dinitrate, isosorbide mononitrate, or combinations thereof, and the anaesthetic agent is selected from the group consisting of lidocaine, prilocaine, tetracaine, prilocaine, dibucaine, benzocaine and combinations thereof.

The use of these combinations of specific agents together with the use of a heat patch providing heating to a temperature of from 50 to 55 degrees Celsius has been found to be particularly effective. This combination results in a significant reduction in the number of attempts required to achieve successful cannulation as well as a perceived ease in cannulation for the medical practitioner.

In one embodiment the weight ratio of organic nitrate vasodilator agent to anaesthetic agent is from 5:1 to 1:5, such as from 4:1 to 1:4 or from 3:1 to 1:3 or from 2:1 to 1:2.

In one embodiment the weight ratio of organic nitrate vasodilator agent to anaesthetic agent is from 3:1 to 1:5, such as from 5:2 to 1:5 or from 4:3 to 1:5. In one embodiment the weight ratio of organic nitrate vasodilator agent to anaesthetic agent is from 2:1 to 1:5, such as from 1:1 to 1:5 or from 2:3 to 1:5. In one embodiment the ratio of organic nitrate vasodilator agent to anaesthetic agent is from 2:1 to 1:4, such as from 1:1 to 1:4 or from 2:3 to 1:4. In one embodiment the ratio of organic nitrate vasodilator agent to anaesthetic agent is from 2:1 to 1:3, such as from 1:1 to 1:3 or from 2:3 to 1:3.

In one embodiment there is either substantially the same amount (by weight) of organic nitrate vasodilator agent and anaesthetic agent, i.e. about a 1:1 ratio, or there is more anaesthetic agent than organic nitrate vasodilator agent (by weight). A reference to "about" or substantially" envisages a tolerance of ±5% such as +2% or ±1%.

In one embodiment the organic nitrate vasodilator agent is glyceryl trinitrate and the anaesthetic agent is lidocaine, prilocaine, tetracaine, or a combination thereof.

The device of the present invention may further comprise one or more additional agents suitable for topical administration.

The types of agent that may optionally be included are, for example, emollients, emulsifiers, thickening agents, buffers, solvents, mixed solvents, preservatives, coloring agents, fragrances, anti-oxidants, preservatives antimicrobial and antifungal actives, anti-inflammatory actives, lower alcohols, polyols, esters of fatty acids, oils, and waxes, silicones, antifoam agents, hydrating agents, stabilizers, surfactants, fillers, sequestrants, anionic, cationic, nonionic and amphoteric polymers, propellants, alkalifying and acidifying agents and mixtures thereof.

The agents that may optionally be included can, for example, be selected from water, polypropylene glycols, saturated and/or unsaturated alkyl alpha hydroxy acids, resins, gums (e.g. guar gum, xanthan gum and the like), waxes (both naturally occurring and synthetic), polymers for aiding the film-forming properties and substantivity of the composition (such as a copolymer of eicosene and vinyl pyrrolidone), skin penetration aids, chelators and sequestrants, and aesthetic components such as fragrances, pigments, colorings, essential oils, skin sensates, astringents, skin soothing agents, skin healing agents and the like. Non-limiting examples of aesthetic components include panthenol and derivatives (e.g. ethyl panthenol), aloe vera, pantothenic acid and its derivatives, clove oil, menthol, camphor, eucalyptus oil, eugenol, menthyl lactate, witch hazel distillate, allantoin, bisabalol, dipotassium glycyrrhizinate and the like.

The device is intended to be sized so that is a localised source of facilitating venepuncture. It is not intended to be sized to cover an entire limb but rather to cover a specific area that is the chosen location for accessing a vein of interest.

In one embodiment, therefore, the lower surface of the device has a surface area of 50cm² or less, such as 45cm² or less, or 40cm² or less, preferably 35cm² or less, such as 30cm² or less.

It is envisaged that the lower surface of the device may suitably have a surface area of from 1 to 50cm², such as from 3 to 45cm², or from 4 to 40cm², preferably from 5 to 35cm², such as from 6 to 30cm². In one embodiment the lower surface of the device has a surface area of from 4 to 50cm², such as from 9 to 45cm², or from 10 to 40cm², preferably from 12 to 35cm², such as from 15 to 30cm².

In one embodiment the lower surface of the device has a maximum dimension (i.e. the longest of its dimensions spanning from one edge to another, e.g. selected from length, width and diameter) that is 15cm or less, preferably 10cm or less, such as 9cm or less or 8cm or less or 7cm or less.

In one embodiment the lower surface of the device has a minimum dimension (i.e. the shortest of its dimensions spanning from one edge to another, e.g. selected from length, width and diameter) that is 0.1cm or more or 0.2cm or more, preferably 0.5cm or more, such as 1cm or more or 1.5cm or more.

The device may have any shape. It may be a shape with a curved outer edge, such as a circle or ellipse, or a shape with straight outer edges, such as a polygon (which may be regular or irregular), or it may have some curved and some straight outer edges (for example it may be a polygon but with one or more of its corners replaced by curves, such as a square or rectangle with curved off corners). It may be a decorative shape, such as a flower or a star or a pillar.

In one embodiment the device has a shape selected from the group consisting of: a circular shape, a triangular shape, quadrilateral shape (e.g. rectangular, square, rhomboidal, trapezoidal or a parallelogram, wherein the corners may optionally be curved off), and an elliptical shape.

In one embodiment the device has a quadrilateral shape (wherein the corners may optionally be curved off) wherein each side of the quadrilateral has a length of from 1 to 10cm, such as from 2 to 8cm.

In one embodiment the device has a circular or elliptical shape with a maximum diameter from 1 to 10cm, such as from 2 to 8cm.

In the device of the present invention the device is configured and arranged such that when the device is applied to the skin of the patient, with the lower surface contacting the skin of the patient, the vasodilator agent and the anaesthetic agent are released from the lower side of the device onto the patient's skin at the location of a vein to be accessed, due to (i) the vasodilator agent and anaesthetic agent each being provided as a coating on the lower surface, or (ii) the device including a reservoir that releasably contains both the vasodilator agent and the anaesthetic agent; or (iii) the device including one reservoir that releasably contains the vasodilator agent and one reservoir that releasably contains the anaesthetic agent. The agents can then be absorbed via the skin.

In one embodiment the vasodilator agent and anaesthetic agent are each provided as a coating on the lower surface. It may be that a composition comprising both the vasodilator agent and the anaesthetic agent is coated on the lower surface. Alternatively the vasodilator agent and the anaesthetic agent may each be provided as a separate coating on the lower surface. The vasodilator agent may be coated first followed by the anaesthetic agent, or vice versa.

In one embodiment the vasodilator agent and the anaesthetic agent are each provided in the form of a topical composition. It may be that a topical composition is provided that includes both the vasodilator agent and the anaesthetic agent or it may be that there is a first topical composition that provides the vasodilator agent and a second topical composition that provides the anaesthetic agent.

It may be that a topical composition comprising both the vasodilator agent and the anaesthetic agent is coated on the lower surface, such that when the lower surface of the device contacts the skin of the patient, the vasodilator agent and the anaesthetic agent are released from the lower side of the device onto the patient's skin at the location of a vein to be accessed.

It may be that a first topical composition comprising the vasodilator agent and a second topical composition comprising the anaesthetic agent are each coated on the lower surface, such that when the lower surface of the device contacts the skin of the patient, the vasodilator agent and the anaesthetic agent are released from the lower side of the device onto the patient's skin at the location of a vein to be accessed.

In one embodiment the or each topical composition is provided in the form of a cream, a gel, an ointment or a paste, which is coated on the lower surface of the device.

In one embodiment the or each topical composition further comprises one or more pharmaceutically acceptable diluent, carrier or excipient.

In one embodiment the device of the present invention comprises from 0.5 g to 10 g of said topical composition or of said first and second topical compositions, such as from 0.5 mg to 5 g, or from 1 g to 5 g or from 1 g to 3 g.

In one embodiment, the amount of organic nitrate vasodilator agent in the topical composition or in the combined first and second topical compositions is 20w/w% or less, such as from 0.01 to 20w/w% or from 0.1 to 20w/w%. In one embodiment, the amount of organic nitrate vasodilator agent in the topical composition or in the combined first and second topical compositions is 10w/w% or less, such as from 0.01 to 10w/w% or from 0.1 to 10w/w%. It may be 8w/w% or less, such as from 0.01 to 8w/w% or from 0.1 to 8w/w%. It may be 6w/w% or less, such as from 0.01 to 6w/w% or from 0.1 to 6w/w%. It may be 5w/w% or less, such as from 0.01 to 5w/w% or from 0.1 to 5w/w%. It may, for example, be from 0.2 to 3w/w% or from 0.5 to 2w/w%.

In one embodiment the amount of anaesthetic agent in the topical composition or in the combined first and second topical compositions is 20w/w% or less, such as from 0.01 to 20w/w% or from 0.1 to 20w/w%. In one embodiment the amount of anaesthetic agent in the topical composition or in the combined first and second topical compositions is 10w/w% or less, such as from 0.01 to 10w/w% or from 0.1 to 10w/w%. It may be 9w/w% or less, such as from 0.01 to 9w/w% or from 0.1 to 9w/w%. It may be 8w/w% or less, such as from 0.01 to 8w/w% or from 0.1 to 8w/w%. It may be 6w/w% or less, such as from 0.01 to 6w/w% or from 0.1 to 6w/w%. It may, for example, be from 0.2 to 5w/w% or from 0.5 to 4w/w%.

The coating may be applied directly to the heat patch, or there may be one or more substrate located between the heat patch and the coating.

The skilled person will be aware of techniques for coating active agents onto a substrate. These techniques can be used to coat the agents directly on to the heat patch or onto a substrate that is connected directly or indirectly to the heat patch.

The skilled person will also be aware of techniques for only releasing coated active agents onto a surface (the skin) at a desired point in time. For example, a release sheet may be provided to cover the coatings until the device is ready to use, and this release sheet is removed before the lower surface is contacted with the patient's skin.

In an alternative embodiment the device includes one or more reservoir. There may be one reservoir that contains both the vasodilator agent and the anaesthetic agent. Alternatively there may be one reservoir that contains the vasodilator agent and one reservoir that contains the anaesthetic agent.

The skilled person will be aware of techniques for providing active agents in a reservoir and for techniques for allowing those agents to be released from the reservoir onto a surface (the skin) at a desired point in time. For example, the reservoir may have an outlet with an opening that extends to the lower surface, and this outlet may be sealable until the device is ready for use. Thus a seal sheet may be provided to prevent the active agent leaving the reservoir via the outlet until the device is ready to use, and this seal is removed or broken before the lower surface is contacted with the patient's skin or is removed or broken by contact with the patient's skin.

In one embodiment the vasodilator agent and the anaesthetic agent are each provided in crystal form.

These crystals of the agents can be coated to the lower surface of the device. The crystals of the agents can be released onto a surface (the patient's skin) at a desired point in time. It may be that the heat generated by the heat patch causes the crystalline structure to be lost and therefore the agents might no longer be crystals when they are released.

These crystals of the agents can be contained in a reservoir of the device. The crystals of the agents can be released from the reservoir onto a surface (the patient's skin) at a desired point in time. It may be that the heat generated by the heat patch causes the crystalline structure to be lost and therefore the agents might no longer be crystals when they are released.

In one embodiment, the device comprises three or more layers. In one such embodiment the device comprises a top layer, which provides the upper surface of the device, a bottom layer, which provides the lower surface of the device, and the heat patch in a layer between the top layer and the bottom layer.

In another embodiment, the device comprises a top layer, which provides the upper surface of the device, and a bottom layer, which provides the lower surface of the device, and the heat patch is located at or near the top layer.

In one embodiment the device of the invention further comprises means for releasably attaching the device to the skin. The device may, for example, be releasably affixed to a patient's skin by an adhesive layer. Such an adhesive layer may cover some, most or all of the lower surface of the device.

In one embodiment, the device may be provided with an adhesive border or one or more adhesive tab located around the perimeter of the lower surface of the device. This border or tab(s) serve to adhere the device to the skin of a patient. Where tabs are used, preferably there are two or more tabs. Preferably the tabs are symmetrically arranged around the perimeter of the lower surface of the device. Preferably the tabs are of a size and number such that they are provided to extend from 50% or more of the perimeter of the lower surface of the device. It will be appreciated that there could, for example, be two or three or four tabs (which might be larger sized tabs) or there could be six or eight or ten tabs (which might be smaller sized tabs).

Adhesives suitable for attaching products to a patient's skin in a releasable manner are well known, for example the adhesives of the type used in sticking plaster products for dressing skin wounds.

It is preferable that the adhesive is non-hydrophilic. It is preferable that the adhesive is a nonaromatic hot melt based adhesive.

The adhesive may be covered with a release sheet to prevent it from curing before use.

The device of the present invention can be activated before placing it on the patient's skin or can be activated once is attached to the patient's skin at the targeted site of insertion.

To use the device, the medical practitioner simply needs to take one of the "one size fits all" devices according to the invention, apply it to the skin of the patient at the location of the selected vein of interest, and activate the device by pressure.

Alternatively, the medical practitioner can take one of the "one size fits all" devices according to the invention, activate the device by pressure, and then apply it to the skin of the patient at the location of the selected vein of interest.

If the device is activated before it is applied to the patient's skin, the device should preferably be applied to the patient's skin within two or three minutes of activation, preferably within 90 seconds of activation, such as from 1 second to 90 seconds after activation, more preferably within a minute of activation, such as from 1 second to 60 seconds after activation.

As noted above, in the method for facilitating intravenous access to a selected vein in a patient by using the device of the invention, the method comprises:
i) placing the device of the invention onto the skin of the patient at a location proximal to the selected vein;
ii) activating the device so as to initiate the exothermic chemical reaction in the heat patch to heat the patch to a temperature of from 50 to 55 degrees Celsius;
   wherein steps i) and ii) can be carried out in any order,
   and then
iii) leaving the device on the skin of the patient for a time period of from one minute to ten minutes;
   and then
iv) removing the device from the patient's skin.

Preferably steps i) and ii) are carried out within three minutes of each other, more preferably within two minutes of each other, such as within 90 seconds of each other, most preferably within a minute of each other.

In one embodiment, the device is activated by being pressed or flexed. It could be that the device is one where there is a crystallization reaction heat patch and the device is activated by applying pressure to the trigger in the heat patch, e.g. pressing or flexing that trigger.

The device is left on the skin of the patient for a time period of from one minute to ten minutes before being removed. Although in theory it could be left on for longer, most heat patches remain hot for a time frame of around 10 minutes or so and the invention is based on the finding of a technical effect from the combination of the 50 to 55 degrees Celsius heating and the active agents. Therefore the surprising efficacy of the invention is achieved by the use of a 1 to 10 minute window.

In one embodiment, the device is left on the skin of the patient for a time period of from one minute to 8 minutes before being removed, e.g. from 1 to 7 minutes or from 1 to 6 minutes or from 2 to 5 minutes.

The invention further provides a kit comprising the device according to the invention and an intravenous access device. These kits may include the device of the invention and one or more intravenous access devices selected from needles, syringes, cannulas, lines or catheters.

The kit may further comprise one or more additional components selected from vials, containers, dressings, alcohol prep swabs/wipes, povidone/iodine swabs/wipes, cotton wool, strapping, gloves, tubes, tape, bandages or gauze sponges.

The term "intravenous access device" as used herein is intended to include any device suitable for administration of medication or fluids and extraction/administration of blood to a patient. Intravenous access devices include but are not limited to needles, cannulas (e.g. peripheral cannula), catheters (e.g. central venous catheter), central venous lines, syringes or infusion sets.

The term "patient" as used herein is intended to include any mammalian subject requiring intravenous access. The term includes human and non-human animals. Thus, the term "patient" includes but is not limited to veterinary patients, such as veterinary mammalian patients. These can include but are not limited to primates, cattle, horses, dogs, cats, guinea pigs, rabbits, rats, and mice.

### Description of the Drawings

The invention can be further understood from the non-limiting drawings, in which:

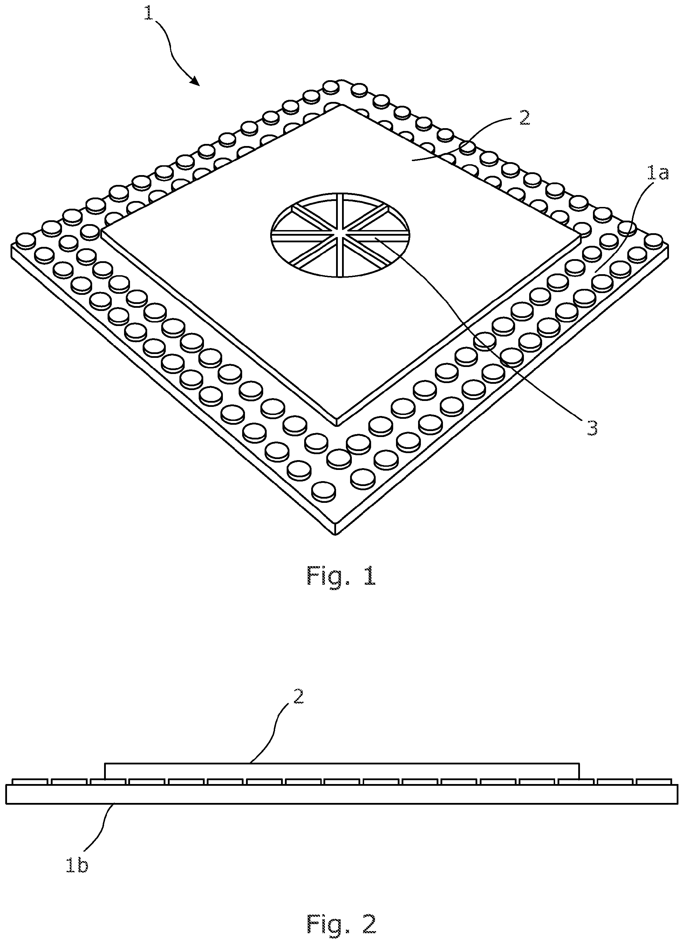

**Figure 1** shows a perspective view of a device according to the invention without a release sheet present.
**Figure 2** shows a side view of the device of Figure 2.
**Figure 3** shows a view from below of the device of Figure 1 when a release sheet is present.
**Figure 4** shows intravenous access devices, one or more of which may be provided together with the device as shown in Figures 1-3 in the form of a kit.

The device 1 is a sterile device which comprises a top layer which provides the upper surface 1a of the device, and a bottom layer, which provides the lower surface 1b of the device.

A heat patch 2 is included in the device, at or near to the upper surface.

The lower surface 1b of the device is coated with about 0.5g of a first composition comprising 2% by weight of glyceryl trinitrate and is further coated with about 0.5g of a second composition that comprises 4% by weight of tretacaine or that comprises 2% by weight lidocaine and 2% by weight prilocaine). It will be appreciated that a single layer could alternatively be used, where this layer is a mixture of the first and second compositions. It will be appreciated that the amounts of composition and the concentrations of active agent therein could also be varied, in accordance with the teachings above regarding the total amount of active agent present.

The heat patch 2 is a crystallization type heat patch comprising a flexible container wherein a supercooled aqueous sodium acetate solution and a trigger are contained. The trigger is a metallic disc 3, such as a stainless steel disc, and is immersed in the solution. The flexible container is made from a material which is not affected by the sodium acetate solution and which is impermeable to the sodium acetate solution and which is stable at temperatures up to and above 55 degrees Celsius, such as a suitable polymeric material.

The heat patch may be of the type described in US 4,077,390.

The device is provided with an adhesive border 4 around the perimeter of the lower surface, which can be used to releasably adhere the device to the skin of a patient, in a similar manner to a sticking plaster product that is used for wound dressings. The border is shown to extend around the entire perimeter of the device, but it will be appreciated that as an alternative it may be in the form of discrete tabs.

The adhesive border 4 is provided with a release sheet 5 that covers the adhesive and prevents it from curing before the device is used. The release sheet is removed before the device is applied to the skin of the patient. The release sheet 5 also covers the layers of composition and prevents release of the agents before the device is used.

Thus in the illustrated embodiment, a single release sheet covers both the adhesive border and the lower surface. However, it will be appreciated that there could be one release sheet for the adhesive and another release sheet for the layers of composition.

Thus when the release sheet 5 is removed, the border 4 of adhesive is exposed on the lower surface of the device, and can be used to temporarily and releasably secure the device to the skin of the patient. The layers of composition are exposed and thus there is release of the agents onto the skin of the patient.

The heat patch 2 is activated such that there is also heating to 50 to 55 degrees C.

The present device is not a pain relief treatment. Instead, the device uses the combination of the organic nitrate vasodilator agent and the amino based anaesthetic agent and the exothermic chemical reaction heats the patch to a temperature of from 50 to 55 degrees Celsius to prepare the skin for cannulation. The device is not designed for treating pain in a subject, whereby prolonged treatment timeframes would be used to ensure pain relief for the subject. Instead, the device provides a short (e.g. 10 minutes or less) preparation treatment that improves the subsequent cannulation process. Unexpectedly, when this preparation treatment is used, the time taken to cannulate and the number of attempts to reach successful cannulation can be significantly reduced. As can be seen from the examples, the results are a notable improvement in terms of the time taken to cannulate and the number of attempts to reach successful cannulation can be significantly reduced

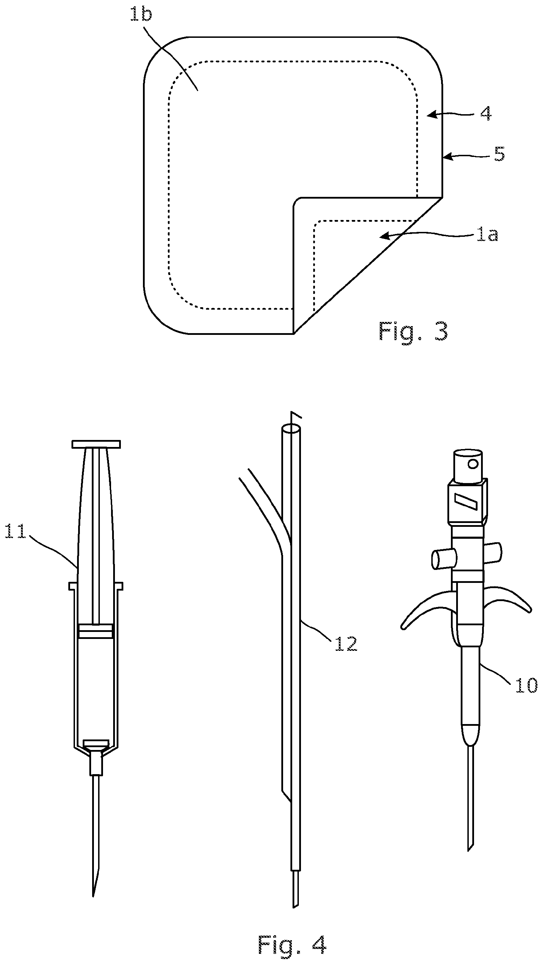

The device 1 is therefore used to prepare the skin for intravenous access, e.g. cannulation, and can be provided in the form of a kit in combination with an intravenous access device. Such access devices are illustrated in Figure 4, which shows a cannula 10, a syringe 11, and a catheter 12.

The invention will now be further described, by means of illustration only, by means of the following non-limiting examples.

### Examples

### Example 1

Example 1 illustrates an exemplary device according to the present invention.

The device is a sterile device which comprises a top layer, which provides the upper surface of the device, a bottom layer, which provides the lower surface of the device, and a heat patch in a layer between the top layer and the bottom layer.

The device is not limited to any particular shape. The device may, for example, have an elliptical or circular shape, or a quadrilateral shape, or any other shape mentioned in the present specification.

The layers are each made of a flexible material. The top and bottom layers are sealed together about their perimeters to hold the heating patch therebetween. The layers can be sealed together using any mechanism known in the art, for example they may be laminated together or secured together with adhesive.

The lower surface of the device is coated with about 0.5g of a first composition comprising 2% by weight of glyceryl trinitrate and is further coated with about 0.5g of a second composition that comprises 4% by weight of tretacaine or that comprises 2% by weight lidocaine and 2% by weight prilocaine). It will be appreciated that a single layer could alternatively be used, where this layer is a mixture of the first and second compositions. It will be appreciated that the amounts of composition and the concentrations of active agent therein could also be varied, in accordance with the teachings above regarding the total amount of active agent present.

The lower surface of the device is provided with a release sheet that covers these layers of composition and prevents release of the agents before the device is used. The release sheet is removed before the device is applied to the skin of the patient.

The heat patch is a crystallization type heat patch comprising a flexible container wherein a supercooled aqueous sodium acetate solution and a trigger are contained. The trigger is a metallic disc, such as a stainless steel disc, and is immersed in the solution. The flexible container is made from a material which is not affected by the sodium acetate solution and which is impermeable to the sodium acetate solution and which is stable at temperatures up to and above 55 degrees Celsius, such as a suitable polymeric material.

The heat patch may be of the type described in US 4,077,390.

The device is provided with an adhesive border around the perimeter of the lower surface, which can be used to releasably adhere the device to the skin of a patient, in a similar manner to a sticking plaster product that is used for wound dressings. The border may extend around the entire perimeter, or may be in the form of discrete tabs.

The adhesive border is provided with a release sheet that covers the adhesive and prevents it from curing before the device is used. The release sheet is removed before the device is applied to the skin of the patient.

It will be appreciated that a single release sheet could cover both the adhesive border and the lower surface.

Prior to carrying out venepuncture, e.g. by a syringe for the withdrawal of blood from a vein, or by cannulation, the release sheets are removed. The device is then attached to the skin of a patient at a location proximal to the selected vein.

The device is activated either just before or just after the device is located on the skin; preferably within a two minute window before or after that steps of locating the device on the skin. This activation comprises applying pressure, such that the trigger initiates crystallisation. The exothermic reaction will produce temperatures in the range of 50 to 55 degrees Celsius and the active agents will contact the skin and be absorbed. The device can be left on the skin for 1 to 10 minutes and then removed.

### Example 2

A study was conducted to evaluate the advantageous effects on patient when a device based on the present invention is used before cannulation. This utilised a layer of a composition comprising 2% by weight of glyceryl trinitrate (Percutol Glyceryl Trinitrate 2% Ointment by Aspire Pharma) and a layer of a composition comprising 2% by weight of lidocaine (Anbesol Gel by Alliance Pharmaceuticals) and a heat patch which generates heat due to crystallisation and which is based on supercooled sodium acetate aqueous solution (ClickHeat by Helios). Once activated by pressure, this heats to 52-54 degrees Celsius with a lower surface temperature of 43 degrees Celsius and it remains hot for up to 10 minutes.

The study included 25 patients with ages between 19and 52 and from different ethnic groups. Two of the patients had body mass indexes (BMI) above 30 which classify them in the obese category. Consent was obtained and a history of allergies/medical conditions was elicited. Conditions noted included hypertension, asthma and eczema.

A junior doctor performed the cannulation process for all of the patients over two sessions; he had three years of experience in cannulation.

For the right hand of each patient cannulation was performed without using the aid of the device of the invention. A tourniquet was applied, starting the process of searching for a vein; timing began from this point to the insertion of a successful cannula. A 22 gauge blue cannula was used.

For the left hand of the patient, a device based on the invention was used to assist cannulation. Once the heat patch was activated the device was applied to the skin of to the left hand and remained there for two minutes before being removed.

A tourniquet was then applied, starting the process of searching for a vein; timing began from this point to the insertion of a successful cannula. A 22 gauge blue cannula was used.

The success in cannulation in each hand was recorded by the junior doctor in terms of whether or not cannulation was achieved. The number of attempts required for successful cannulation (up to a maximum of three), and the perceived ease of cannulation (easy/moderate/difficult) was also recorded for every attempt in each hand.

The time in seconds that was taken from applying the tourniquet up to the insertion of a successful cannula was also recorded, and a comparison was made between the time for each patient in relation to the left hand and right hand to see the extent to which using the device .

### Results

| | Successful | Unsuccessful |
|---|---|---|
| Success of cannulation with the device | 96% | 4% |
| Success of cannulation without the device | 88% | 12% |

The three subjects in whom cannulation was unsuccessful (no cannulation after three attempts) in one or both hands were excluded from the remaining data.

| | Success on 1st attempt | Success on 2nd attempt |
|---|---|---|
| Attempts before successful cannulation with the device | 86.3% | 13.6% |
| Attempts before successful cannulation without the device | 72.7% | 27.2% |

The time taken for cannula insertion showed that in 77.2% of the patients the time taken to achieve successful cannulation was shorter when using the device of the invention than when not using the device.

The doctor conducting the procedure reported that when using the device, cannulation was easy in 72.7%, moderate in 22.7% and difficult in 4.5%. Without the device, cannulation was reported as easy in 59%, moderate in 27.2% and difficult in 13.6% of patients.

## Claims

1. A device for application to the skin of a patient to facilitate intravenous access, the device having an upper surface and a lower surface, wherein the lower surface is suitable for contacting the skin of the patient at the location of a vein to be accessed,
the device comprising a heat patch, an organic nitrate vasodilator agent and an amino based anaesthetic agent which is an amino ester or amino amide,
wherein the heat patch is activated by the application of pressure and in this regard can be flexed or pressed to initiate an exothermic chemical reaction to heat the patch to a temperature of from 50 to 55 degrees Celsius;
wherein the device is configured and arranged such that in use the device can be activated by the application of pressure to the patch so as to initiate the exothermic chemical reaction in the heat patch which heats the patch to a temperature of from 50 to 55 degrees Celsius for a period of time of one minute or more;
and wherein the device is configured and arranged such that when the device is applied to the skin of the patient, with the lower surface contacting the skin of the patient, the vasodilator agent and the anaesthetic agent are released from the lower side of the device onto the patient's skin at the location of a vein to be accessed, due to (i) the vasodilator agent and anaesthetic agent each being provided as a coating on the lower surface, or (ii) the device including a reservoir that releasably contains both the vasodilator agent and the anaesthetic agent; or (iii) the device including one reservoir that releasably contains the vasodilator agent and one reservoir that releasably contains the anaesthetic agent.

2. The device of claim 1 wherein the heat patch:
(a) is capable of an exothermic oxidation reaction and comprises a chemical composition that is exothermically reactive in the presence of air; or
(b) is capable of an exothermic crystallization reaction and comprises a container that contains a supercooled aqueous salt solution which, when activated, releases heat.

3. The device of claim 1 wherein the exothermic chemical reaction is:
a) a crystallization of a supercooled aqueous salt solution; or
b) a crystallization of a sodium acetate solution.

4. The device of claim 3, wherein the heat patch comprises a trigger which is a metal or mineral strip or disc, which initiates the exothermic chemical reaction when pressed or flexed.

5. The device of any one of claims 1 to 4 wherein:
a) the vasodilator agent and anaesthetic agent are each provided as a coating on the lower surface and a release sheet is provided to cover the coatings until the device is ready to use; or
b) the device includes a reservoir that releasably contains both of the vasodilator agent and the anaesthetic agent, wherein the reservoir has an outlet with an opening that extends to the lower surface, and this outlet is sealable until the device is ready for use; or
c) the device includes one reservoir that releasably contains the vasodilator agent and one reservoir that releasably contains the anaesthetic agent, wherein each reservoir has an outlet with an opening that extends to the lower surface, and this outlet is sealable until the device is ready for use.

6. The device of any one of claims 1 to 5 wherein the organic nitrate vasodilator agent is selected from the group consisting of: glyceryl trinitrate, isosorbide dinitrate, isosorbide mononitrate, erythritol tetranitrate, pentaerythritol tetranitrate, and combinations thereof.

7. The device of any one of claims 1 to 6 wherein the organic nitrate vasodilator agent:
a) is present in an amount of from 1 mg to 50 mg; or
b) is present in an amount of from 3 mg to 30 mg; or
c) is present in an amount of from 5mg to 20mg.

8. The device of any one of claims 1 to 7 wherein the amino ester or amino amide anaesthetic agent is selected from the group consisting of: procaine, chloroprocaine, tetracaine, cocaine, benzocaine, dibucaine, lidocaine, mepivacaine, prilocaine, bupivacaine, levobupivacaine, ropivacaine, articaine, and etidocain, and combinations thereof.

9. The device of any one of claims 1 to 8 wherein the amino ester or amino amide anaesthetic agent:
a) is present in an amount of from 3 mg to 75 mg; or
b) is present in an amount of from 5 mg to 60 mg; or
c) is present in an amount of from 10 mg to 40 mg.

10. The device of any one of claims 1 to 9 wherein the lower surface of the device has a surface area of:
a) 50cm² or less; or
b) 35cm² or less.

11. The device of any one of claims 1 to 10 wherein when the heat patch is activated to initiate the exothermic chemical reaction:
a) the lower surface of the device is, as a consequence, heated to a temperature of from 40 to 46 degrees Celsius; and/or
b) the heat patch is heated to a temperature of from 50 to 55 degrees Celsius heat for a period of time of less than 15 minutes, such as from one minute to ten minutes.

12. The device of any one of claims 1 to 11 wherein the device comprises (i) a topical composition, provided as a coating on the lower surface, that includes both the vasodilator agent and the amino based anaesthetic agent or (ii) a first topical composition, provided as a coating on the lower surface, that provides the vasodilator agent and a second topical composition, provided as a coating on the lower surface, that provides the amino based anaesthetic agent.

13. The device of claim 12 wherein the amount of organic nitrate vasodilator agent in the topical composition or in the combined first and second topical compositions is:
a) 20w/w% or less; or
b) from 0.01 to 20w/w%; or
c) from 0.1 to 20w/w%; or
d) 8 w/w% or less; or
e) from 0.01 to 8w/w%; or
f) from 0.1 to 8w/w%.

14. The device of any one of claims 12 to 13 wherein the amount of amino based anaesthetic agent in the topical composition or in the combined first and second topical compositions is:
a) 20w/w% or less; or
b) from 0.01 to 20w/w%; or
c) from 0.1 to 20w/w%; or
d) 8 w/w% or less; or
e) from 0.01 to 8w/w%; or
f) from 0.1 to 8w/w%.

15. The device of any one of claims 1 to 14 wherein the weight ratio of organic nitrate vasodilator agent to anaesthetic agent is:
(a) from 3:1 to 1:5; or
(b) from 2:1 to 1:5.

16. A kit comprising the device as defined in any one of claims 1-15 and an intravenous access device, wherein the intravenous access device is optionally selected from needles, syringes, cannulas and catheters.

## Patentansprüche

1. Vorrichtung zum Aufbringen auf die Haut eines Patienten zum Erleichtern von intravenösem Zugang, wobei die Vorrichtung eine obere Oberfläche und eine untere Oberfläche aufweist, wobei die untere Oberfläche zum Kontakt mit der Haut des Patienten an dem Ort einer Vene, zu der zugegangen werden soll, geeignet ist,
wobei die Vorrichtung ein Wärmepflaster, einen organischen Nitrat-Vasodilator und ein Anästhetikum auf Aminobasis, das ein Aminoester oder Aminoamid ist, umfasst,
wobei das Wärmepflaster durch die Anwendung von Druck aktiviert wird und hierfür gebogen oder gedrückt werden kann, um eine exotherme chemische Reaktion zum Erwärmen des Pflasters auf eine Temperatur von 50 bis 55 Grad Celsius auszulösen;
wobei die Vorrichtung so gestaltet und angeordnet ist, dass die Vorrichtung in Verwendung durch die Anwendung von Druck auf das Pflaster aktiviert werden kann, um die exotherme chemische Reaktion in dem Wärmepflaster auszulösen, die das Pflaster für einen Zeitraum von einer Minute oder mehr auf eine Temperatur von 50 bis 55 Grad Celsius erwärmt;
und wobei die Vorrichtung so gestaltet und angeordnet ist, dass, wenn die Vorrichtung auf die Haut eines Patienten aufgebracht wird, wobei die untere Oberfläche mit der Haut des Patienten in Kontakt steht, der Vasodilator und das Anästhetikum von der Unterseite des Vorrichtung an dem Ort einer Vene, zu der zugegangen werden soll, auf die Haut des Patienten freigesetzt wird, da (i) der Vasodilator und das Anästhetikum jeweils als eine Beschichtung an der unteren Oberfläche bereitgestellt sind, oder (ii) die Vorrichtung ein Reservoir aufweist, das sowohl den Vasodilator als auch das Anästhetikum freigebbar enthält; oder (iii) die Vorrichtung ein Reservoir, das den Vasodilator freigebbar enthält, und ein Reservoir, das das Anästhetikum freigebbar enthält, aufweist.

2. Vorrichtung gemäß Anspruch 1, wobei das Wärmepflaster:
(a) zu einer exothermen Oxidationsreaktion fähig ist und eine chemische Zusammensetzung umfasst, die in Gegenwart von Luft exotherm reaktionsfähig ist; oder
(b) zu einer exothermen Kristallisationsreaktion fähig ist und einen Behälter umfasst, der eine unterkühlte wässrige Salzlösung enthält, die, wenn aktiviert, Wärme freigibt.

3. Vorrichtung gemäß Anspruch 1, wobei die exotherme chemische Reaktion ist:
a) eine Kristallisation einer unterkühlten wässrigen Salzlösung; oder
b) eine Kristallisation einer Natriumacetatlösung.

4. Vorrichtung gemäß Anspruch 3, wobei das Wärmepflaster einen Auslöser umfasst, der ein(e) Metalloder Mineralstreifen oder -scheibe ist, der/die, wenn gedrückt oder gebogen, die exotherme chemische Reaktion auslöst.

5. Vorrichtung gemäß einem der Ansprüche 1 bis 4, wobei:
a) der Vasodilator und das Anästhetikum jeweils als eine Beschichtung auf der unteren Oberfläche bereitgestellt sind und eine Ablösefolie bereitgestellt ist, um die Beschichtungen zu bedecken, bis die Vorrichtung zur Verwendung bereit ist; oder
b) die Vorrichtung ein Reservoir aufweist, das sowohl den Vasodilator als auch das Anästhetikum freigebbar enthält, wobei das Reservoir einen Auslass mit einer Öffnung aufweist, die zu der unteren Oberfläche verläuft, und dieser Auslass verschließbar ist, bis die Vorrichtung zur Verwendung bereit ist; oder
c) die Vorrichtung ein Reservoir, das den Vasodilator freigebbar enthält, und ein Reservoir, das das Anästhetikum freigebbar enthält, aufweist, wobei jedes Reservoir einen Auslass mit einer Öffnung aufweist, die zu der unteren Oberfläche verläuft, und dieser Auslass verschließbar ist, bis die Vorrichtung zur Verwendung bereit ist.

6. Vorrichtung gemäß einem der Ansprüche 1 bis 5, wobei der organische Nitrat-Vasodilator ausgewählt ist aus der Gruppe bestehend aus: Glyceryltrinitrat, Isosorbiddinitrat, Isosorbidmononitrat, Erythritoltetranitrat, Pentaerythritoltetranitrat und Kombinationen davon.

7. Vorrichtung gemäß einem der Ansprüche 1 bis 6, wobei der organische Nitrat-Vasodilator:
a) in einer Menge von 1 mg bis 50 mg vorhanden ist; oder
b) in einer Menge von 3 mg bis 30 mg vorhanden ist; oder
c) in einer Menge von 5 mg bis 20 mg vorhanden ist.

8. Vorrichtung gemäß einem der Ansprüche 1 bis 7, wobei das Aminoester- oder Aminoamid-Anästhetikum ausgewählt aus der Gruppe bestehend aus: Procain, Chlorprocain, Tetracain, Cocain, Benzocain, Dibucain, Lidocain, Mepivacain, Prilocain, Bupivacain, Levobupivacain, Ropivacain, Articain und Etidocain und Kombinationen davon.

9. Vorrichtung gemäß einem der Ansprüche 1 bis 8, wobei das Aminoester- oder Aminoamid-Anästhetikum:
a) in einer Menge von 3 mg bis 75 mg vorhanden ist; oder
b) in einer Menge von 5 mg bis 60 mg vorhanden ist; oder
c) in einer Menge von 10 mg bis 40 mg vorhanden ist.

10. Vorrichtung gemäß einem der Ansprüche 1 bis 9, wobei die untere Oberfläche der Vorrichtung eine Oberfläche aufweist von:
a) 50 cm² oder weniger; oder
b) 35 cm² oder weniger.

11. Vorrichtung gemäß einem der Ansprüche 1 bis 10, wobei, wenn das Wärmepflaster aktiviert wird, um die exotherme chemische Reaktion auszulösen:
a) die untere Oberfläche der Vorrichtung als Folge auf eine Temperatur von 40 bis 46 Grad Celsius erwärmt wird; und/oder
b) das Wärmepflaster für einen Zeitraum von weniger als 15 Minuten, wie z. B. von einer Minute bis zehn Minuten, auf eine Temperatur von 50 bis 55 Grad Celsius Wärme erwärmt wird.

12. Vorrichtung gemäß einem der Ansprüche 1 bis 11, wobei die Vorrichtung (i) eine topische Zusammensetzung, die als eine Beschichtung auf der unteren Oberfläche bereitgestellt ist, die sowohl den Vasodilator als auch das Anästhetikum auf Aminobasis enthält, oder (ii) eine erste topische Zusammensetzung, die als eine Beschichtung auf der unteren Oberfläche bereitgestellt ist, die den Vasodilator bereitstellt, und eine zweite topische Zusammensetzung, die als eine Beschichtung auf der unteren Oberfläche bereitgestellt ist, die das Anästhetikum auf Aminobasis bereitstellt, umfasst.

13. Vorrichtung gemäß Anspruch 12, wobei die Menge an organischem Nitrat-Vasodilator in der topischen Zusammensetzung oder in der kombinierten ersten und zweiten topischen Zusammensetzung beträgt:
a) 20 Gew./Gew.-% oder weniger; oder
b) von 0,01 bis 20 Gew./Gew.-%; oder
c) von 0,1 bis 20 Gew./Gew.-%; oder
d) 8 Gew./Gew.-% oder weniger; oder
e) von 0,01 bis 8 Gew./Gew.-%; oder
f) von 0,1 bis 8 Gew./Gew.-%.

14. Vorrichtung gemäß einem der Ansprüche 12 bis 13, wobei die Menge an Anästhetikum auf Aminobasis in der topischen Zusammensetzung oder in der kombinierten ersten und zweiten topischen Zusammensetzung beträgt:
a) 20 Gew./Gew.-% oder weniger; oder
b) von 0,01 bis 20 Gew./Gew.-%; oder
c) von 0,1 bis 20 Gew./Gew.-%; oder
d) 8 Gew./Gew.-% oder weniger; oder
e) von 0,01 bis 8 Gew./Gew.-%; oder
f) von 0,1 bis 8 Gew./Gew.-%.

15. Vorrichtung gemäß einem der Ansprüche 1 bis 14, wobei das Gewichtsverhältnis von organischem Nitrat-Vasodilator zu Anästhetikum beträgt:
(a) von 3:1 bis 1:5; oder
(b) von 2:1 bis 1:5.

16. Kit, umfassend die Vorrichtung gemäß einem der Ansprüche 1-15 und eine intravenöse Zugangsvorrichtung, wobei die intravenöse Zugangsvorrichtung gegebenenfalls ausgewählt ist aus Nadeln, Spritzen, Kanülen und Kathetern.

## Revendications

1. Dispositif destiné à être appliqué sur la peau d'un patient pour faciliter l'accès intraveineux, le dispositif ayant une surface supérieure et une surface inférieure, la surface inférieure étant appropriée pour entrer en contact avec la peau du patient à l'emplacement d'une veine à laquelle il faut accéder,
le dispositif comprenant un patch thermique, un agent vasodilatateur de type nitrate organique et un agent anesthésique à base d'amino qui est un amino ester ou un amino amide,
le patch thermique étant activé par l'application de pression et, à cet égard, pouvant être fléchi ou pressé pour initier une réaction chimique exothermique afin de chauffer le patch à une température de 50 à 55 degrés Celsius ;
le dispositif étant configuré et agencé de telle sorte que, lors de l'utilisation, le dispositif peut être activé par l'application de pression sur le patch de manière à initier la réaction chimique exothermique dans le patch thermique qui chauffe le patch à une température de 50 à 55 degrés Celsius pendant une période de temps d'une minute ou plus ;
et le dispositif étant configuré et agencé de telle sorte que lorsque le dispositif est appliqué sur la peau du patient, avec la surface inférieure en contact avec la peau du patient, l'agent vasodilatateur et l'agent anesthésique sont libérés de la face inférieure du dispositif sur la peau du patient à l'emplacement d'une veine à laquelle il faut accéder, en raison du fait que (i) l'agent vasodilatateur et l'agent anesthésique sont chacun fournis sous la forme d'un revêtement sur la surface inférieure, ou (ii) le dispositif comprend un réservoir qui contient de manière libérable à la fois l'agent vasodilatateur et l'agent anesthésique ; ou (iii) le dispositif comprend un réservoir qui contient de manière libérable l'agent vasodilatateur et un réservoir qui contient de manière libérable l'agent anesthésique.

2. Dispositif selon la revendication 1, le patch thermique :
(a) étant capable d'une réaction d'oxydation exothermique et comprenant une composition chimique qui est exothermiquement réactive en présence d'air ; ou
(b) étant capable d'une réaction de cristallisation exothermique et comprenant un récipient qui contient une solution saline aqueuse surfondue qui, lorsqu'elle est activée, libère de la chaleur.

3. Dispositif selon la revendication 1 la réaction chimique exothermique étant :
a) une cristallisation d'une solution saline aqueuse surfondue ; ou
b) une cristallisation d'une solution d'acétate de sodium.

4. Dispositif selon la revendication 3, le patch thermique comprenant un déclencheur qui est une bande ou un disque métallique ou minéral, qui initie la réaction chimique exothermique lorsqu'il est pressé ou fléchi.

5. Dispositif selon l'une quelconque des revendications 1 à 4,
a) l'agent vasodilatateur et l'agent anesthésique étant chacun fournis sous la forme d'un revêtement sur la surface inférieure et une feuille détachable étant fournie pour recouvrir les revêtements jusqu'à ce que le dispositif soit prêt à être utilisé ; ou
b) le dispositif comprenant un réservoir qui contient de manière libérable à la fois l'agent vasodilatateur et l'agent anesthésique, le réservoir ayant une sortie avec une ouverture qui s'étend jusqu'à la surface inférieure, et cette sortie pouvant être fermée hermétiquement jusqu'à ce que le dispositif soit prêt à être utilisé ; ou
c) le dispositif comprenant un réservoir qui contient de manière libérable l'agent vasodilatateur et un réservoir qui contient de manière libérable l'agent anesthésique, chaque réservoir ayant une sortie avec une ouverture qui s'étend jusqu'à la surface inférieure, et cette sortie pouvant être fermée hermétiquement jusqu'à ce que le dispositif soit prêt à être utilisé.

6. Dispositif selon l'une quelconque des revendications 1 à 5, l'agent vasodilatateur de type nitrate organique étant choisi dans le groupe constitué par : le trinitrate de glycéryle, le dinitrate d'isosorbide, le mononitrate d'isosorbide, le tétranitrate d'érythritol, le tétranitrate de pentaérythritol, et des combinaisons de ces derniers.

7. Dispositif selon l'une quelconque des revendications 1 à 6, l'agent vasodilatateur de type nitrate organique,
a) étant présent en une quantité de 1 mg à 50 mg ; ou
b) étant présent en une quantité de 3 mg à 30 mg ; ou
c) étant présent en une quantité de 5 mg à 20 mg.

8. Dispositif selon l'une quelconque des revendications 1 à 7, l'agent anesthésique de type amino ester ou amino amide étant choisi dans le groupe constitué de : procaïne, chloroprocaïne, tétracaïne, cocaïne, benzocaïne, dibucaïne, lidocaïne, mépivacaïne, prilocaïne, bupivacaïne, lévobupivacaïne, ropivacaïne, articaïne et étidocaïne, et des combinaisons de ces derniers.

9. Dispositif selon l'une quelconque des revendications 1 à 8, l'agent anesthésique de type amino ester ou amino amide,
a) étant présent en une quantité de 3 mg à 75 mg ; ou
b) étant présent en une quantité de 5 mg à 60 mg ; ou
c) étant présent en une quantité de 10 mg à 40 mg.

10. Dispositif selon l'une quelconque des revendications 1 à 9, la surface inférieure du dispositif ayant une surface de :
a) 50 cm² ou moins ; ou
b) 35 cm² ou moins.

11. Dispositif selon l'une quelconque des revendications 1 à 10, lorsque le patch thermique est activé pour initier la réaction chimique exothermique,
a) la surface inférieure du dispositif est, en conséquence, chauffée à une température de 40 à 46 degrés Celsius ; et/ou
b) le patch thermique est chauffé à une température de 50 à 55 degrés Celsius pendant une période de temps inférieure à 15 minutes, telle qu'une minute à dix minutes.

12. Dispositif selon l'une quelconque des revendications 1 à 11, le dispositif comprenant (i) une composition topique, fournie sous forme de revêtement sur la surface inférieure, qui comprend à la fois l'agent vasodilatateur et l'agent anesthésique à base d'amino ou (ii) une première composition topique, fournie sous forme de revêtement sur la surface inférieure, qui fournit l'agent vasodilatateur et une seconde composition topique, fournie sous forme de revêtement sur la surface inférieure, qui fournit l'agent anesthésique à base d'amino.

13. Dispositif selon la revendication 12, la quantité d'agent vasodilatateur de type nitrate organique dans la composition topique ou dans les première et seconde compositions topiques combinées étant de :
a) 20 % en poids ou moins ; ou
b) de 0,01 à 20 % en poids ; ou
c) de 0,1 à 20 % en poids ; ou
d) 8 % en poids ou moins ; ou
e) de 0,01 à 8 % en poids ; ou
f) de 0,1 à 8 % en poids.

14. Dispositif selon l'une quelconque des revendications 12 à 13, la quantité d'agent anesthésique à base d'amino dans la composition topique ou dans les première et seconde compositions topiques combinées étant de :
a) 20 % en poids ou moins ; ou
b) de 0,01 à 20 % en poids ; ou
c) de 0,1 à 20 % en poids ; ou
d) 8 % en poids ou moins ; ou
e) de 0,01 à 8 % en poids ; ou
f) de 0,1 à 8 % en poids.

15. Dispositif selon l'une quelconque des revendications 1 à 14, le rapport pondéral d'agent vasodilatateur de type nitrate organique à l'agent anesthésique étant :
(a) de 3:1 à 1:5 ; ou
(b) de 2:1 à 1:5,

16. Kit comprenant le dispositif selon l'une quelconque des revendications 1 à 15 et un dispositif d'accès intraveineux, le dispositif d'accès intraveineux étant éventuellement choisi parmi des aiguilles, des seringues, des canules et des cathéters.
